# EUROPEAN PATENT APPLICATION

(11) **EP 3 153 184 A2**
(43) Date of publication of application: **12.04.2017**
(21) Application number: 16189173.4
(22) Date of filing: 16.09.2016
(51) Int. Cl.: A61L 2/20, C01B 11/02, A61L 9/14, C02F 1/76

(54) **METHOD AND SYSTEM FOR DISINFECTING WITH CHLORINE DIOXIDE GAS**

(30) Priority: 16.09.2015 BE 201500231; 27.10.2015 BE 201505694
(71) Applicant: Aqua Ecologic, 9000 Gent (BE)
(72) Inventor: Daneels, Rik, 8870 Izegem (BE); Loncke, Trees, 8870 Izegem (BE)
(74) Representative: Brants, Johan P.E.

(57) **Abstract**

The present invention relates to a method for disinfecting a room with chlorine dioxide gas, comprising the following steps: generating chlorine dioxide gas in a reaction cell; releasing said chlorine dioxide gas from said reaction cell; and transferring the released chlorine dioxide gas to the room to treat.

## Description

### TECHNICAL FIELD

The present invention relates to a method and system for disinfecting and/or sterilizing a room and/or apparatus placed in a room by means of chlorine dioxide gas.

### BACKGROUND

Chlorine dioxide is historically typically used as a bleaching agent in the paper and pulp industry, but is also used as a biocide or oxidant, for example for water treatment or odour control. Chlorine dioxide has a relatively low oxidation potential compared to other oxidants, but has nevertheless a high oxidation capacity. Moreover, when used as a disinfectant, it forms less by-products compared to chlorine.

In order to meet stability requirements, chlorine dioxide is preferably produced in batches. To this end, WO 2015/136478 describes an aqueous chlorine dioxide composition with a concentration of at least 4 gram chlorine dioxide per litre, a method for preparing such aqueous composition and a kit containing concentrated solutions of chlorite salt and bisulphate and persulphate salt.

However, many production methods have limitations as reagents and/or by-products possibly are noxious for the room to disinfect and/or the product to disinfect. Therefore, chlorate is preferably not used in the food industry and corrosive agents can have an impact on metals, synthetic materials, electronics, measuring devices or medical apparatus. Therefore, the possible use of chlorine dioxide technology is largely limited, and mainly for applications in which chlorine dioxide is extremely useful. Thus, there is a need for new ways to release chlorine dioxide with a high purity in the room to treat without however reducing the generation efficiency.

The present invention aims to offer a solution for one or more of the said problems or defects.

### SUMMARY

To this end, the invention provides a method and a system for disinfecting a room with chlorine dioxide.

To this end, the invention provides in a first aspect a method for disinfecting a room with chlorine dioxide gas, comprising the steps:
- generating chlorine dioxide gas in a reaction cell;
- releasing said chlorine dioxide gas from said reaction cell; and
- transferring the released chlorine dioxide gas in the room to treat.

More specifically, the generated chlorine dioxide gas is guided through a gas-permeable membrane before being released in the room to treat. In this way, possibly noxious agents can be stopped partially or completely by means of the gas-permeable membrane, so that damage to the room to disinfect can be suppressed partially and preferably completely.

In a second aspect, the present invention provides a system for disinfecting a room with chlorine dioxide gas, comprising a reaction cell for generating and/or storing chlorine dioxide gas, a dispersing unit in fluidic connection with said reaction cell for dispersing the chlorine dioxide gas in said reaction cell in a room to treat and a gas-permeable membrane placed between said reaction cell and said dispersing unit. This offers the advantage that chemical agents that can possibly damage apparatus in the room to treat, can be stopped by said gas-permeable membrane.

In a third aspect, the present invention provides the use of a method according to the first aspect of the invention for disinfecting a room in which apparatus, such as electronic devices, are placed. This offers the advantage that the used technology does not have a negative impact on the apparatus.

### DESCRIPTION OF THE FIGURES

The explicit characteristics, advantages and objectives of the present invention will become clear for the skilled worker in the technical domain of the invention from the following detailed description of the embodiment of the invention and the attached figures. The figures are only illustrative of the invention, and are in no case limitative of the invention.
Figure 1 shows a first system for dosing a purified chlorine dioxide gas by means of a gas-permeable membrane 36.
Figure 2 shows a second system for dosing a purified chlorine dioxide gas by means of a gas-permeable membrane 36.

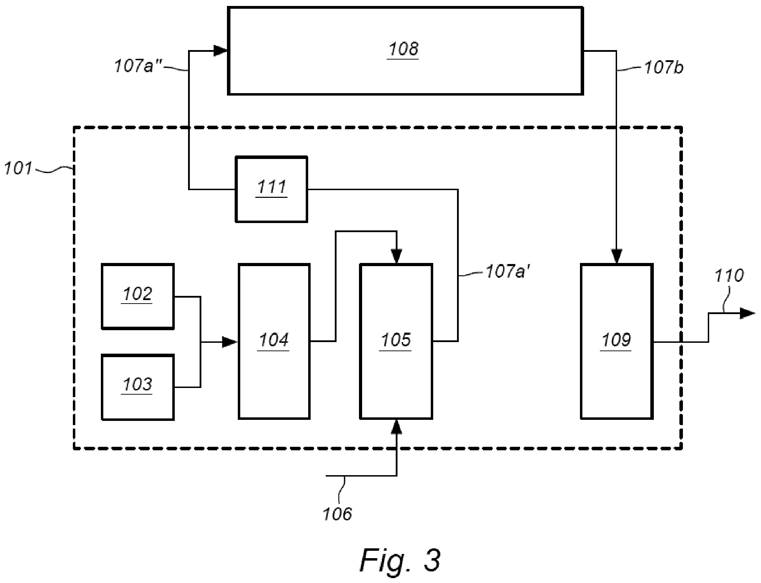

Figure 3 is a schematic illustration of a system for disinfecting a room.

### DETAILED DESCRIPTION OF THE INVENTION

Unless otherwise specified, all terms used in the description of the invention, including technical and scientific terms, shall have the meaning as they are generally understood by the worker in the technical field of the invention. For a better understanding of the description of the invention, the following terms are explained specifically.

When "approximately" or "about" are used in the document together with a measurable quantity, a parameter, a period or moment, etc., variations of +/-20% or less, preferably +/-10% or less, more preferably +/-5% or less, still more preferably +/-1% or less, and even still more preferably +/-0.1% or less than and of the cited value are meant, as far as such variations apply to the invention that is described. It will however be clearly understood that the value of the quantity at which the term "approximately" or "about" is used, is itself specified.

The citation of numeric intervals by means of end points includes all integers, fractions and/or real numbers between the end points, including these end points.

The term "disinfecting" is used as a synonym for the term "decontaminating" or "purifying" and refers to at least partially destroying one of more types of kind of plagues, pathogens or germs of a disease. Preferably, at least 90% of said pathogens or germs of a disease are destroyed, more preferably at least 97% and most preferably 100%. The term "kind of plague" is meant as a synonym of the term "noxious organism" and refers to each organism that is present unwantedly or that has a negative effect on a human, animal, plant and/or the environment. Examples of kinds of plagues are weed, micro-organisms, pathogens, fungi, larvae, insects, parasites, nematodes, algae, mites, rodents, bacteria, viruses, etc. The term "pathogen" or "pathogens" is used as a synonym for the term "pathogenic organism" and refers to several bacteria, viruses, fungi, yeasts and protozoa that can cause disease and/or death of a human, animal, plant or other biological organism. Pathogenic spores are spores that are produced by a pathogen. Specific examples of pathogens that produce spores, include, but are not limited to, members of the genera *Bacillus, Clostridium, Desulfotomaculans, Sporolactobacillus,* and *Sporpsarcina,* members of the *Phylum Apicomplexa* (such as *Plasmodium falciparum* and *Cryptosporidium parvum*), and phytopathogenic fungi. In the context of the present invention, the term "disinfecting" is also meant as "sterilizing". When sterilising, a higher degree of killing of pathogens is obtained compared to disinfecting: degree 'log 6' when sterilising compared to degree 'log 5' when disinfecting. The sterilisation of a room is mainly carried out in applications where a high degree of hygiene is required, such as for example in rooms for food treatment or production, in medical rooms such as for example in hospitals, as well as in veterinary applications.

The term "chlorine dioxide" or "ClO₂" refers to a molecule indicated with the CAS number 10049-04-4 and appears as a gas at standard pressure and temperature. Chlorine dioxide has a greenish, yellow colour with a characteristic odour similar to chlorine and is a particularly efficient biocide that kill pathogens, such as bacteria, viruses and parasites, fast and efficiently. Chlorine dioxide gas molecules can also kill atomized germs of a disease, and can also spread through tears and cracks in an article or a building or a room and thus reach each surface that is possibly contaminated with a germ of a disease. Chlorine dioxide is very well water-soluble, but compared to chlorine, it does not react with water. It appears in an aqueous solution as a dissolved gas. Chlorine dioxide is recognised as oxidative, possibly explosive, corrosive, toxic and environmentally dangerous. The boiling point of chlorine dioxide is 9.7°C at standard pressure.

The term "sodium chlorite" refers to a chemical molecule with the gross formula NaClO₂ and is indicated with the CAS number 7758-19-2. The term "sodium bisulphate" refers to a chemical molecule with the gross formula NaHSO₄ and is indicated with the CAS number 7681-38-1. The term "sodium persulphate" refers to a chemical molecule with the gross formula Na₂S₂O₈ and is indicated with the CAS number 7775-27-1. In an alternative embodiment, lithium, potassium, rubidium, cesium and/or francium are used instead of sodium. This offers the advantage that counter-ions interfere minimally with the active agents in the aqueous compositions and thus do not jeopardize the obtained chlorine dioxide composition.

The term "reaction cell" is meant as a reaction vessel in which solid, liquid and/or gaseous agents can be loaded and discharges. Preferably, said reaction cell contains an aqueous solution in which chemical reactions can take place.

### Method

In a first aspect, the invention provides a method for disinfecting a room with chlorine dioxide gas, comprising the steps:
- generating chlorine dioxide gas in a reaction cell;
- releasing said chlorine dioxide gas from said reaction cell; and
- transferring the released chlorine dioxide gas to the room to treat.

Chlorine dioxide can, in the context of the present invention, be generated via known methods according to the state of the art. Specific examples of combinations of reagents that can be used, include, but are not limited to: sodium chlorate, sodium chloride and sulphuric acid; sodium chlorate and hydrochloric acid; sodium chlorate, sodium chlorite, sodium chloride and sulphuric acid; sodium chlorate, sodium chlorite and hydrochloric acid; sodium chlorate, sulphur dioxide and sulphuric acid; sodium chlorate, methanol and sulphuric acid; sodium chlorite and chlorine; sodium chlorite and hydrochloric acid and/or sulphuric acid; sodium chlorite, oxidizing gas and sulphuric acid; sodium chlorate, sodium chloride, hydrogen peroxide and/or methanol and sulphuric acid; sodium chlorite, sodium hypochlorite and hydrochloric acid and/or sulphuric acid; and sodium chlorate, glucose and sulphuric acid. Other suitable combinations of reagents can also be used, such as for example electrochemical reactions in which chlorine dioxide is generated from sodium chlorite or sodium chlorate; or photochemical reactions in which chlorine dioxide is generated from sodium chlorite or sodium chlorate under influence of UV radiation, e.g. at 254 nm. Such methods are amongst other things described in WO 2005/016011, US 4 874 489 and US 8 652 411.

Preferably, chlorine dioxide gas is generated in said reaction cell as a result of the reaction of chlorite with an activator in an aqueous solution. Thereto, in an aqueous solution, an alkali chlorite, shortly chlorite, is added to - and optionally stirred with an activator such as preferably bisulphate; bisulphate and persulphate; citric acid; or a combination of one or more of said agents. The use of an agitator when mixing reagents in said aqueous solution is preferably avoided. Usually, other acids can also be used as an activator.

The release of said chlorine dioxide gas from said aqueous solution can take place by spontaneous diffusion of said chlorine dioxide gas in said aqueous solution to the gas phase above said aqueous solution. Subsequently, the chlorine dioxide gas can be brought into the aqueous solution. Alternatively, said chlorine dioxide gas can be released from said aqueous solution by purging the aqueous solution with air or nitrogen gas as a carrier gas. This purging is also referred to with the term "stripping", referring to the physical separation process in which one or more agents are removed from a liquid and included in the gas or vapour stream which was brought into contact with said liquid.

The transfer of the released chlorine dioxide gas to the room to treat can be carried out by guiding the chlorine dioxide gas and eventually the carrier gas in which the outlet ends in the room to treat.

More specifically, the invention provides a method for disinfecting a room with chlorine dioxide gas, in which the released chlorine dioxide gas is guided through a gas-permeable membrane before being released to the room to treat.

In this way, possibly noxious agents can be stopped partially or completely by means of the gas-permeable membrane, so that damage to the room to disinfect can be suppressed partially and preferably completely. The presence of chlorate ions in mist drops is for example not desirable, specifically for disinfecting medical rooms. Chlorate can be present as contamination in the chlorite raw material, but can also be formed as a by-product during the production of chlorine dioxide from chlorite, especially when in an acid environment and/or at increased temperature. Chlorate ions from mist drops can namely precipitate in the room and on the present apparatus. However, chlorate is considered as a noxious agent for the human body, and the presence of chlorate in the environment should therefore be avoided. Moreover, the presence of corrosive agents in the reaction mixture before creating chlorine dioxide gas causes corrosion of the apparatus in the room to treat. Any appropriate construction material can be used for the membrane, as long as the membrane is sufficiently porous to allow the flow of gases and sufficiently hydrophobic to prevent the passage of the aqueous solution. An appropriate gas-permeable membrane comprises an expanded polytetrafluoroethylene plate. The membrane can be provided as a composite with carrying or supporting materials to offer the structural strength that is needed in use. Such supporting materials can be selected from a diversity of polymer materials, such as for example, but not limited to, polyvinyl chloride and polyethylene, and other materials, such as glass fibre tissues, felt and films. The pore size of the membrane can vary strongly, depending on the desired flow rate of chlorine dioxide through the membrane. The pore size cannot be so small that the chlorine dioxide gas flow is prevented through the pores and can moreover not be so big that the liquid flow through the membrane becomes possible. Said pore size is preferably comprised between 0.001 µm and 15 µm. The porosity of the membrane can vary strongly, also depending on the desired flow rate of chlorine dioxide through the membrane. Considerations with respect to the membrane strength also dictate the chosen porosity. In general, the porosity of the membrane varies from about 50% to about 98%. The thickness of the membrane is determined by the strength of the chosen material. The thickness of the carrying membrane varies between about 0.1 and about 2.0 mm. It is not essential for the present invention that the membrane is made of hydrophobic material throughout the thickness, as the surface of the membrane oriented towards an aqueous solution in the reaction cell is hydrophobic and as a result, the flow of the aqueous medium through the membrane can be prevented. The membrane can close off the discharge of the reaction cell in any desired geometrical form, in general flat form or in a tubular form.

In a preferred embodiment, the present invention provides a method according to the first aspect of the invention, in which said room is closed off partially or completely before disinfection. This offers the advantage that no or only a limited amount of chlorine dioxide gas and/or possibly present noxious agents can leak to the environment, as a result of which the effective concentration of chlorine dioxide in the room to disinfect remains maximal and there is no danger of undesired contact between said chlorine dioxide and/or possible present noxious agents and adjacent or contiguous rooms and the apparatus present in these rooms. Avoiding or at least significantly reducing the leaking of chlorine dioxide gas to contiguous rooms contributes to the safety of both professional operators and persons in the environment of the room to disinfect. Preferably, said room is closed off by means of a substantially gas-impermeable closure. A closed room such as a closed article, a closed room or a sealed building should be understood as an environment in which substantially all fluidic connections with the environment are or were closed off, for example by means of plastic screens or other sheets, tape, isolation, sealants or combinations of that, and in which preferably, said screens are gas-impermeable. In a further embodiment, said closed room is made of a 'glove bag', a 'gas bag', an 'air bag' or an 'atmosbag'. Said closed room is preferably also provided with one or more in- and/or outlets allowing specific agents to be moved in and/or out of the closed room.

In a preferred embodiment, the present invention provides a method according to the first aspect of the invention, in which the chlorine dioxide gas is brought into the aqueous solution in the gas phase, for example by purging the aqueous solution with air, consequently is brought through a gas-permeable membrane and finally is released in the room to treat. Preferably, after the passage through the gas-permeable membrane, the chlorine dioxide gas is not absorbed in a second aqueous solution any more, but it is brought directly to the room to treat. As a result, the chlorine dioxide in the aqueous solution is released with an optimal efficiency in the room to treat, while at the same time, mist drops with chlorate ions as well as other possibly noxious agents are not or only strongly reduced released into the room.

In a preferred embodiment, the present invention provides a method according to the first aspect of the invention, in which said gas-permeable membrane has a pore size of less than 5.0 µm, preferably less than 2.5 µm. This offers the advantage that mist drops can be stopped by the gas-permeable membrane while gas molecules can diffuse without much resistance through the membrane. More preferably, said membrane has a pore size of less than 1.0 µm and still more preferably a pore size between 1 nm and 1000 nm. Most preferably, said membrane has a pore size between 5 nm and 500 nm, such as for example between 10 nm, 20 nm, 50 nm, 75 nm, 100 nm, 125 nm, 150 nm, 200 nm and 250 nm or any value between these values. Gas-permeable membranes with a too small pore size lead to a high pressure drop over the membrane as a result of which more energy is needed to transfer the chlorine dioxide through the membrane.

The term "aqueous solution" should be understood as an aqueous reaction medium in which reagents for the synthesis of chlorine dioxide gas can be brought. Reagents can contain chlorite and chlorate, as well as one or more activators.

In a preferred embodiment, the present invention provides a method according to the first aspect of the invention, in which de molar ratio chlorite : activator is lower than 4:1, and preferably about 2:1. A lower molar ratio chlorite : activator leads to a higher efficiency for the conversion of chlorite to chlorine dioxide. Said ratio chlorite : activator is preferably higher than 1:100, more preferably higher than 1:25 and still more preferably higher than 1:10. A too low molar ratio chlorite : activator leads to a slower activation of the chlorite.

In a preferred embodiment, the present invention provides a method according to the first aspect of the invention, in which said activator is a liquid or a solid at standard pressure and temperature. This offers the advantage that the activator has a low damp pressure, as a result of which the presence of the activator in the gas phase above the aqueous solution of chlorine dioxide is relatively low at standard pressure and temperature. This offers the advantage that little or no activator with the chlorine dioxide in the gas phase is released into the room to treat. This is particularly advantageous when the activator has possibly toxic or noxious characteristics with respect to the environment or the apparatus in the room to treat. Hydrogen chloride, which is an activator for chlorite, will for example diffuse easily through the gas-permeable membrane and will have a corrosive effect on materials in the room to treat.

In a preferred embodiment, the present invention provides a method according to the first aspect of the invention, in which said activator is a solid at standard pressure and temperature. A solid as an activator offers the advantage with respect to a liquid that the damp pressure is lower at standard pressure and temperature, as a result of which the presence of the activator in the chlorine dioxide gas phase is lower. Thus, possible damage to the room to disinfect is avoided.

In a preferred embodiment, the present invention provides a method according to the first aspect of the invention, in which said activator in acid form is an acid that does not dissociate in water and an acid gas at standard pressure and temperature. Activators such as for example bisulphate will become more acid in an acid environment to for example sulphuric acid, which spontaneously dissociates in water and O₃. The O₃ gas can then spontaneously go to the gaseous phase above the aqueous chlorine dioxide solution and diffuse through the gas membrane to subsequently be released into the room to treat. After reaction with water in the air of the room to treat, sulphuric acid can again be formed, which has a corrosive effect on the apparatus, and is also irritating and noxious for living organisms. Other examples of activators or additives to avoid are amongst other things bicarbonate.

Said activator is preferably an acid, such as an organic acid or an inorganic acid, which can bring the pH of the aqueous solution within the pH range of 2 to 6, and preferably 3 to 5. The appropriate pH offers an optimal working area for activating chlorite. Preferably, said organic acid is a solid. Organic hydroxy carbon acids, such as for example - but not limited to - salicylic acid, lactic acid, acetyl salicylic acid and citric acid, are namely advantageous as they are good activators for generating chlorine dioxide. Most preferably, citric acid is used as an activator. Citric acid offers the advantage that it is highly soluble in water. As a result, a concentration of citric acid up to 500 gram per litre can easily be made and a highly concentrated, acid solution can be added to the aqueous reaction medium for obtaining the appropriate acidity. This offers the advantage that no citric acid precipitation is left behind in the reaction vessel, in the pipes and control valves of the system or on the gas-permeable membrane. Such precipitation would hinder the good operation of the chlorine dioxide generation system and of the gas-permeable membrane and eventually require more maintenance. Because smaller volumes of citric acid can be added, less waste products are moreover generated in the fumigation system.

In a further aspect, the present invention provides a method for making an aqueous solution of chlorine dioxide in which chlorite and/or chlorate are activated by means of an activator. The activator can be selected from the group of acids, preferably organic acids and more preferably citric acid; bisulphate and/or persulphate; and hydrogen peroxide; or a mixture of two or more activators such as most preferably citric acid and hydrogen peroxide. This offers the advantage that a concentrated, stable solution of chlorine dioxide is obtained. The solution can subsequently be used for water treatment or disinfection applications.

In a preferred embodiment, the present invention provides a method according to the first aspect of the invention, in which said chlorine dioxide gas from said aqueous solution is released by purging the aqueous solution with air. Air from the room to disinfect is preferably used to purge the aqueous solution with chlorine dioxide. Thus, chlorine dioxide can be stripped with an optimal efficiency out of said aqueous solution and can be swept away with the gas stream. The air flow rate for purging is preferably comprised between 0.001 m³/h per litre aqueous solution and 1.000 m³/h per litre aqueous solution. The air flow rate is thus partially dependent on the total amount of water. More preferably, said air flow rate is comprised between 0.005 m³/h per litre aqueous solution and 0.200 m³/h per litre aqueous solution, and still more preferably between 0.01 m³/h per litre aqueous solution and 0.10 m³/h per litre aqueous solution. An optimal air flow rate through the aqueous solution leads to an optimal air flow rate through the gas-permeable membrane and is thus also responsible for the good operation of said gas-permeable membrane. As an alternative for air, nitrogen gas can also be used for purging the aqueous chlorine dioxide solution under the same conditions as illustrated above.

In a preferred embodiment, the present invention provides a method according to the first aspect of the invention, in which chlorite is activated by said activator in the presence of hydrogen peroxide. Hydrogen peroxide ensures the fast conversion of chlorate ions to chlorite ions. Thus, the release of chlorate ions, whether or not using a gas-permeable membrane, in the room to treat can be strongly suppressed. For some applications, such as mainly the food industry, chlorite is considered as a contamination that should be avoided and/or remedied. More preferably, chlorite is activated by citric acid in the presence of hydrogen peroxide. In an alternative embodiment, chlorite is activated by persulfate and/or bisulphate in the presence of hydrogen peroxide. In an additional embodiment, the hydrogen peroxide is *in situ* generated.

In a preferred embodiment, the present invention provides a method according to the first aspect of the invention, in which hydrogen peroxide and chlorite are mixed in a molar ratio of about 3:1, such as any ratio between 5:1 and 1:1.

In a preferred embodiment, the present invention provides a method according to the first aspect of the invention, in which chlorite is dosed in said aqueous environment in an amount of more than 0.5 % in weight, preferably more than 1.0 % in weight. This offers the advantage that the total amount in the aqueous reaction medium is smaller, leading to a higher efficiency of the release of chlorine dioxide gas in the room to treat.

In a preferred embodiment, the present invention provides a method according to the first aspect of the invention, in which the room to treat is previously conditioned at a relative humidity of 50% to 85%, preferably 55% to 80% and more preferably 60% to 70%. More preferably, said room is previously conditioned at a relative humidity of 61%, 62%, 63%, 64%, 65%, 66%, 67%, 68%, 69% of 70%, or any value between these values. Lower values of relative humidity offer the advantage that at a particular environmental temperature, less water is present in the air. The gaseous agents with corrosive characteristics that diffuse through the membrane, will, at lower relative humidity, be included slower in mist drops and will consequently not precipitate or precipitate less onto the apparatus in the room to disinfect.

In a preferred embodiment, the present invention provides a method according to the first aspect of the invention, in which the room to treat is previously conditioned at a temperature of 15°C to 35°C, preferably 20°C to 30°C. Most preferably, said room is conditioned at 21°C, 22°C, 23°C, 24°C, 25°C, 26°C, 27°C, 28°C, 29°C of 30°C. The use of higher temperatures than 30°C ensures that possibly present corrosive agents in the release chlorine dioxide gas have a more corrosive attitude in the room to treat. The use of lower temperatures than 15°C is possible, but leads to more difficulties to keep the relative humidity sufficiently low. Preferably, said method is carried out at a temperature above the boiling point of chlorine dioxide, such as for example at a temperature higher than 10°C at standard pressure.

In a preferred embodiment, the present invention provides a method according to the first aspect of the invention, in which remaining amounts of chlorine dioxide gas in the treated room are removed after disinfection. This can for example be realised by means of ventilation or repeated ventilation of the treated room. Alternatively, the chlorine dioxide gas can be neutralised. The term "neutralise" refers to the chemical neutralisation of a chemical agent, preferably by means of chemisorption, physisorption and/or decomposition. As a result of the neutralisation, the concentration of chlorine dioxide in said gas stream is substantially reduced. Said chlorine dioxide can for example be neutralised by means of, preferably intensive, contact with an aqueous solution comprising 10% in weight of NaOH and 10% in weight of sodium thiosulfate hydrate. Preferably, said chlorine dioxide is neutralised in a gas washer. In an alternative or preferably additional embodiment, a filter with a fixed chlorine dioxide adsorbant, preferably active coal, is used for adsorbing remaining chlorine dioxide in the disinfected room. Preferably, said filter is appropriate for treating an air flow rate higher than 1000 m³/hour, preferably between 2000 m³/hour and 10000 m³/hour, more preferably between 2500 m³/hour and 5000 m³/hour and most preferably an air flow rate of 2500, 2750, 3000, 3250, 3500, 3750 or 4000 m³/hour or any value between these values. This offers the advantage that the room to disinfect can be disposed of possibly noxious chlorine dioxide. Preferably, the neutralisation in said room lasts less than 2 hours, more preferably less than 1 hour, more preferably between 5 minutes and 30 minutes and most preferably 5 minutes, 10 minutes, 15 minutes, 20 minutes, 25 minutes or 30 minutes, or any value between these values.

### System

In a second aspect, the present invention offers a system for disinfecting a room with chlorine dioxide gas, comprising a reaction cell for generating and/of stocking chlorine dioxide gas, a dispersing unit in fluidic connection with said reaction cell for dispersing the chlorine dioxide gas in said reaction cell in a room to treat and a gas-permeable membrane placed between said reaction cell and said dispersing unit.

This offers the advantage that chemical agents that can possibly damage apparatuses in the room to treat can be stopped by said gas-permeable membrane.

Figure 5 is a schematic illustration of a system according to the present invention with a decontamination device 101 comprising a stock solution of chlorite 102 and a stock solution comprising a mixture of activator 103, a batch reactor 104 for mixing both stock solutions 102 and 103, a container and/or strip reactor 105 for the temporary storage of an aqueous solution of chlorine dioxide and/or the stripping of chlorine dioxide from said solution by means of a carrier gas, preferably supplied air 106, and one or more fluidic connections 107a', 107a" for guiding a gaseous effluent of said aqueous solution to a room to disinfect 108. Finally, the remaining amount of chlorine dioxide is discharged after the treatment via one or more fluidic connections 107b to an absorption and/or adsorption unit 109 for reducing the amount of chlorine dioxide in the effluent gas 110 before discharge of the effluent gas in the atmosphere. The fluidic connection 107 preferably comprises close to the outlet of the container/strip reactor 105 a gas-permeable membrane 111 for selectively letting pass the chlorine dioxide gas. In an alternative, but simple configuration, the batch reactor 104 and the container and/or strip reactor 105 can be provided as one reaction vessel.

In a perforated embodiment, the present invention provides said system, comprising one or more storage vessels (12, 13) for at least temporarily storing reagents, at least a reaction vessel (14), and at least a chlorine dioxide vessel (15); a pipe network with one or more pumps for moving gases and/or fluids through said pipe network, in which said pipe network is configured for transferring reagents to a reaction vessel, for transferring a mixture in said reaction vessel to a chlorine dioxide vessel and for dispensing chlorine dioxide. In this respect, the internal volume of said pipe network is smaller than the total volume of said chlorine dioxide vessel. Preferably, the internal volume of said pipe network is 50% smaller than the total volume of said chlorine dioxide vessel, and more preferably 80% smaller. Most preferably, the volume of said pipe network is 90%, 92%, 94%, 96%, 98%, 99% smaller than the volume of said chlorine dioxide vessel.

In a preferred embodiment, the present invention provides said system, comprising a chlorine dioxide vessel with a fluid sensor for detecting the fluid level in said chlorine dioxide vessel. In this way, the remaining volume of chlorine dioxide composition in said chlorine dioxide vessel can be monitored. When the remaining volume has decreased below a predefined value, a new batch of chlorine dioxide composition according to the first aspect of the invention can be made via a control system.

In a preferred embodiment, the present invention provides said system, additionally provided with an air pump and ventilation compartments for purging air through said solution comprising chlorine dioxide. More preferably, said ventilation compartments are for purging air through said solution comprising chlorine dioxide provided as a stripping tower, in which said stripping tower is preferably provided with an active height between 25 cm and 200 cm, and preferably equal to 25 cm, 50 cm, 75 cm, 100 cm, 110 cm, 120 cm, 130 cm, 140 cm or 150 cm, or any value between these values. Also preferably, said stripping tower has a height-to-diameter ratio higher than 2:1, preferably higher than 5:1, and most preferably higher than 10:1. Still preferably, said stripping tower has a carrier gas flow rate, preferably an air flow rate, between 250 and 10000 l per minute, preferably between 400 and 4000 l per minute, still more preferably between 800 and 2000 l per minute and most preferably a flow rate of 800, 1000, 1200, 1400, 1600, 1800 or 2000 l per minute or any value between these values. Still preferably, said stripping tower has a fluid recirculation flow rate between 10 l per hour and 1000 I per hour, preferably between 20 l per hour and 500 l per hour. The skilled worker will know that the optimal fluid recirculation flow rate will depend on the volume of the room to disinfect and will be, for smaller rooms, between about 20 l per hour, and, for larger rooms, between 150 and 500 l per hour and more preferably between 200 and 400 l per hour. In a specific embodiment, said fluid recirculation flow rate is equal to 0 l per hour. Such embodiment is feasible in situations in which chlorine dioxide gas spontaneously goes from the fluid phase to the gaseous phase of the reactor.

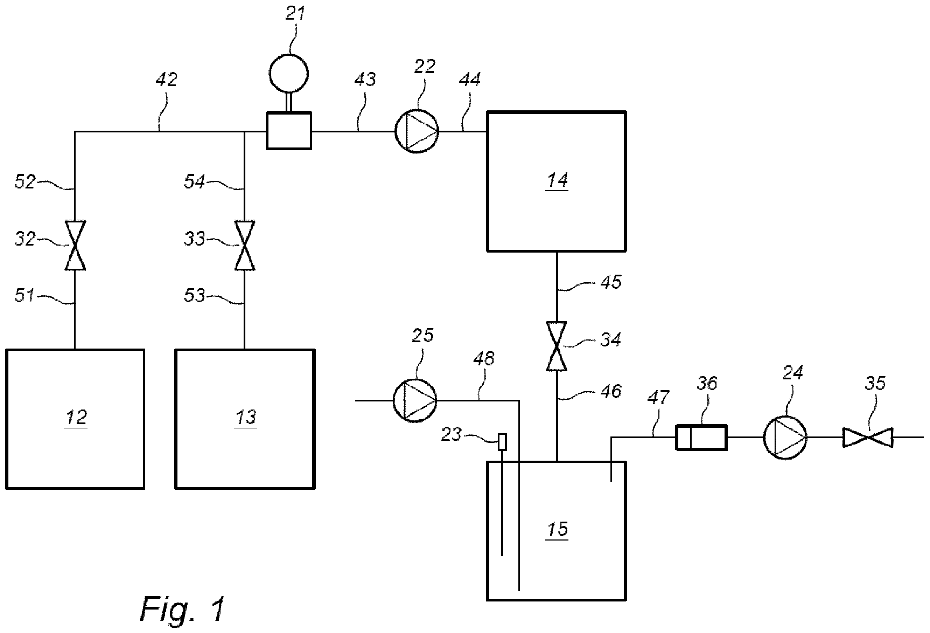

Figure 1 shows a system for preparing a stable chlorine dioxide composition according to the invention. The system comprises a first 12 and a second 13 storage vessel for reagents, such as for example respectively a NaClO₂ solution and a solution comprising an activator, such as for example citric acid or sodium bisulphate and/or sodium persulphate, and optionally hydrogen peroxide. Supply of reagents can be controlled by means of closure valves 32 and 33, respectively, by means of pump 22. The use of a pump becomes optional in cases in which water from a water pipe on pressure is used. In this way, reagents can be transferred to a reaction vessel 14, in which both reagents are brought into contact with each other during the reaction time. After this reaction time, the chlorine dioxide solution is ready for use, and it is transferred to a chlorine dioxide vessel 15. In a more simple configuration, reaction vessel 14 and chlorine dioxide vessel 15 form one physical vessel in which both the mixing and the reacting of reagents takes place, as well as where chlorine dioxide gas is stored and later stripped from the reaction medium, specifically from the aqueous solution. From the aqueous solution of chlorine dioxide in chlorine dioxide vessel 15, the aqueous chlorine dioxide solution can be purged by means of air pump 25 and air pipe 48. The concentrated chlorine dioxide gases can then be filtered by means of a gas-permeable membrane 36 before being dosed in the room to treat by means of a flow rate pump 24 and control valve 35. This offers the advantage that possibly corrosive agents have no noxious effect on flow rate pump 24 and control valve 35.

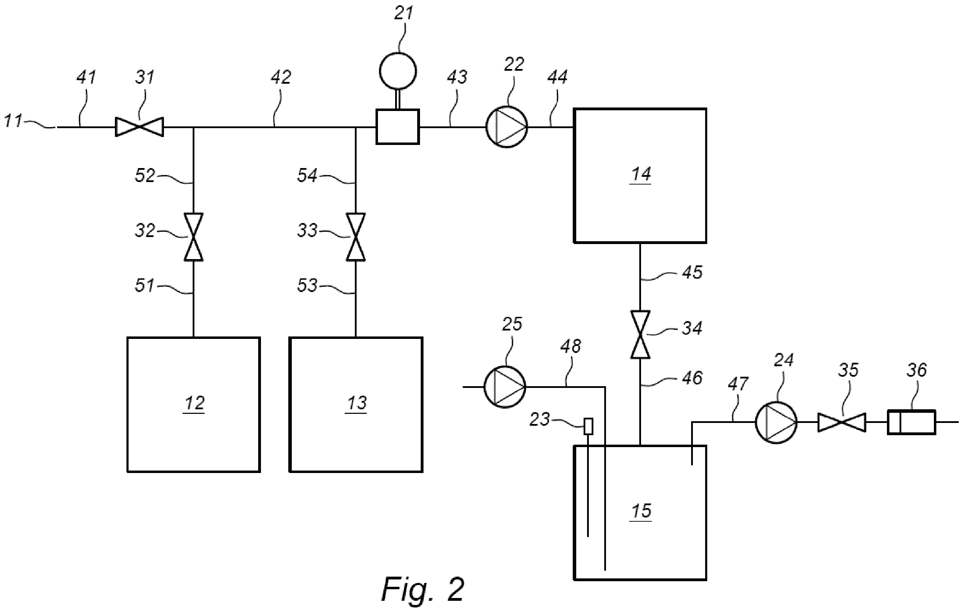

Figure 2 shows a system for preparing a stable chlorine dioxide composition according to the invention. In general, NaClO₂ solution can be used in any concentration. In general, any chemical agent in solution or mixture of chemical agents in solution can be used, that activate sodium chlorite in order to at least partially convert to chlorine dioxide. Examples of such activators include, but are not limited to, sodium bisulphate, sodium persulphate, citric acid, hydrogen peroxide, ... or combinations thereof. The concentration of chlorine dioxide of the prepared stable chlorine dioxide solution can e.g. lie between 1 g/l and 10 g/l, according to amperometric determinations.

In a first preferred embodiment, NaClO₂ solution is used in a concentration that is proportionally higher than the concentration NaClO₂ used at the start of the reaction for the formulation of the chlorine dioxide solution. In said case, the desired sodium chlorite concentration in the reaction mixture in the reaction vessel is obtained by adding water to the reaction vessel. In a second advantageous embodiment, NaClO₂ solution is used in the desired proportional concentration that is required to obtain the desired concentration of NaClO₂ at the start of the reaction for the formulation of the chlorine dioxide solution. In said case, no water must thus be added any more.

The reaction time for the formulation of the stable chlorine dioxide solution depends on the chosen precursors, the concentrations of the chosen precursors in the reaction mixture and the required stability in the time. It is evident that the present invention is not limited to the specified precursors, concentrations of precursors in the reaction mixture or stability.

The system comprises a first 12 and a second 13 storage vessel for reagents, such as for example a NaClO₂ solution and a solution comprising sodium bisulphate and sodium persulphate. These storage vessels are in connection to the main pipe 42 via the fluid connection 51-52 and 53-54. Supply of reagents can be controlled by means of closure valves 32 and 33, respectively. Although not indicated in the figure, the closure valves 32 and 33 are preferably provided with a serially connected fluid pump to control the fluid transport of reagents in the storage vessels 12 and 13 to the main pipe 42. Alternatively, the storage vessels can be provided with a pressure mechanism for applying a pre-pressure to the fluids in the storage vessels.

The main pipe 42 is, at the supply side 41, connected to a water pipe 11 and the water supply can be controlled by means of the closure valve 31. At the discharge side, the main pipe 42 is provided with a flow rate meter 21 and a pump 22. The pump brings the fluid mixture to a pressure of 5 bar. Water and reagents in storage vessels 12 and 13 are guided via the fluid pipe 43-44, flow rate meter 21 and fluid pump 22 to a reaction vessel 14, where the reagents are in contact with each other during the reaction time.

In a practical methodology, chlorine dioxide is formulated in a process consisting of five steps. In a first step, a first volume of water of the water pipe 11 is transferred to a reaction vessel 14. In a second step, sodium chlorite from a storage vessel 12 is transferred to said reaction vessel 14. In a third step, a second volume of water of the water pipe 11 is transferred to a reaction vessel 14. In a fourth step, an aqueous mixture of activator, such as for example citric acid, or sodium bisulphate and/or sodium persulfphate, and optionally hydrogen peroxide from a storage vessel 13 is transferred to said reaction vessel 14. In a last step, a third volume of water of the water pipe 11 is transferred to a reaction vessel 14.

After the reaction, the content of the reaction vessel 14 is transferred to a chlorine dioxide vessel 15 via the transfer pipe 45-46, which is controlled by means of a closure valve 34 and possibly is provided with a fluid pump to stimulate the fluid transfer.

The chlorine dioxide vessel 15 is provided with a fluid sensor 23 which is activated when the content of the chlorine dioxide vessel 15 has decreased below a predetermined level. At a fluid level below a predetermined value, for example lower than 8 litre, the fluid sensor 23 will send a signal to a control system, which is configured to control the adjustable closure valves 31, 32 and 33 and the pump 22 in order to formulate a new volume of chlorine dioxide composition. Moreover, the chlorine dioxide vessel 15 is connected to an air supply pipe 48 and air pump 25 for purging the aqueous chlorine dioxide solution in reaction vessel 15. Via pipe 47, concentrated chlorine dioxide gases can be dosed, possibly regulated by means of a chlorine dioxide pump 24 with injection valve 35. Before being released into the room to treat, the concentrated chlorine dioxide gas is filtered by means of a gas-permeable membrane 36.

In a third aspect, the invention provides a use of a method according to the first aspect of the invention for disinfecting a room in which apparatus, such as for example electric and/or electronic apparatus, are provided.

### EXAMPLES

The invention will now be further described by means of the following examples, without being limited thereto.

### EXAMPLE 1

Chlorine diode gas is formulated from an aqueous solution comprising chlorite as a source for chlorine dioxide, persulphate as an oxidant for the conversion of chlorite to chlorine dioxide and hydrogen peroxide for the conversion of chlorate to chlorite. To an amount of 18 L water, 1 L 25 (m/v)-% NaClO₂ in an aqueous solution is added and 1 L 20 (m/v)-% Na₂S₂O₈ and 5 (m/v)-% H₂O₂ in an aqueous solution. Persulphate causes oxidation of chlorite and ensures a good stability of the obtained chlorine dioxide composition. Hydrogen peroxide allows the conversion of chlorate in the chlorite raw material. The conversion of chlorite to chlorate is complete within a period of 12 hours and the chlorine dioxide concentration is stable at a value of 7000 mg/L ± 100 mg/L during a period of at least 30 days. The reaction progress is summarised in Table 1.

**Table 1. Reaction progress for conversion of chlorite to chlorine dioxide according to example 1, values expressed in mg/L.**

| Reaction time | ClO₂⁻ | ClO₃⁻ | ClO₂ |
|---|---|---|---|
| 12 hours | 0 | 0 | 6900 |
| 3 days | 0 | 0 | 7100 |
| 10 days | 0 | 0 | 6950 |
| 30 days | 0 | 0 | 6925 |

The obtained chlorine dioxide composition is thus very pure in chlorine dioxide gas and comprises few or no volatile by-products, such as for example hydrogen chloride.

After complete conversion of chlorite, the obtained chlorine dioxide composition is used as a stable solution of chlorine dioxide and can thus be used in disinfection applications. In an alternative embodiment, after complete conversion of chlorite, the obtained chlorine dioxide composition is used for the release of chlorine dioxide for fumigation applications. In a first step, the room to treat is conditioned at a relative humidity of about 65%. By choosing a lower relative humidity compared to the usual humidity according to the state of the art, the chlorine dioxide treatment is not experienced as corrosive, as a result of which the room to treat is not affected unnecessarily. The relative humidity is subsequently maintained for a period of about 10 minutes, so that mist drops in the room can dissolve and accordingly, the treatment is less corrosive with respect to apparatus in the room to treat. In a second step, the formulated chlorine dioxide gas is transported over a gas-selective membrane with a pore size of 1000 nm. In order to stimulate the release of chlorine dioxide gas from the aqueous composition, the reaction mixture is purged with air. The room to treat is subsequently released to chlorine dioxide in a release regime of 720 ppm (vol.).h. This means that the room can be exposed during a period of 1 hour at 720 ppm (vol.) or during a period of 2 hour at 350 ppm (vol.). In the room, during the disinfection cycle, one or more ventilators are arranged in order to allow a good spreading of the chlorine dioxide. In a last step, chlorine dioxide is removed from the room to treat up to a concentration of chlorine dioxide smaller than 0,1 ppm (vol.). By removing chlorine dioxide, the room to treat is re-used again quicker.

### EXAMPLE 2

Method according to example 1, in which to an amount of 17.8 L water, 1 L 25 (m/v)-% NaClO₂ in an aqueous solution is added and 1.2 L 20 (m/v)-% Na₂S₂O₈ en 5 (m/v)-% H₂O₂ in an aqueous solution. The conversion of chlorite to chlorate is complete within a period of 12 hours and the chlorine dioxide concentration is stable at a value of 7500 mg/L ± 100 mg/L during a period of at least 30 days.

### EXAMPLE 3

Method according to example 1, in which to an amount of 18 L water, 1 L 25 (m/v)-% NaClO₂ in an aqueous solution is added and 1 L 22.5 (m/v)-% Na₂S₂O₈ en 5 (m/v)-% H₂O₂ in an aqueous solution. The conversion of chlorite to chlorate is complete within a period of 12 hours and the chlorine dioxide concentration is stable at a value of 7500 mg/L ± 100 mg/L during a period of at least 30 days.

### EXAMPLE 4

Chlorine gas is formulated according to the method of example 1, in which bisulphate, persulphate and hydrogen peroxide are added to an aqueous solution of chlorite. Bisulphate allows a quick conversion of chlorite to chlorine dioxide. Persulphate ensures a good stability of the obtained chlorine dioxide composition. Hydrogen peroxide allows the conversion of residual amounts of chlorate in the chlorite raw material and of chlorate formulated by oxidation of chlorite by means of bisulphate to chlorine dioxide.

### EXAMPLE 5

Chlorine dioxide gas is formulated according to the method of example 1, in which citric acid and hydrogen peroxide are added to an aqueous solution of chlorite. Citric acid allows the conversion of chlorite to chlorine dioxide. Hydrogen peroxide allows the conversion of residual amounts of chlorate in the chlorite raw material and of chlorate formulated by oxidation of chlorite by means of bisulphate to chlorine dioxide.

### EXAMPLE 6

Chlorine dioxide gas is formulated from an aqueous solution comprising chlorite as a source of chlorine dioxide and persulphate-bisulphate as an oxidant and stabiliser, respectively. Alternatively, an acid such as for example citric acid can be used as an oxidant. In the case of oxidation of chlorite, chlorine dioxide is generated within a period of about 5 hours. In the presence of bisulphate, the chlorine dioxide solution remains stable for about 30 days. The reaction composition for a solution comprising 0.45% in weight of chlorite is shown in Table 2.

**Table 2. Reaction progress for conversion of chlorite to chlorine dioxide according to comparative example 1, values expressed in mg/L.**

| Reaction time | ClO₂⁻ | ClO₃⁻ | ClO₂ |
|---|---|---|---|
| 1 hours | 1100 | 823 | 3441 |
| 5 hours | 0 | 650 | 4040 |
| 3 days | 0 | 558 | 4753 |
| 30 days | 0 | 0 | 4344 |

During the oxidation of chlorite, chlorate is formulated, which is converted to chlorine dioxide after further oxidation. However, the complete conversion of chlorate to chlorine dioxide lasts more than 15 days, which complicates a practical use of the chlorine dioxide composition for some applications, such as for example in the food industry where chlorate is considered as a contaminant. The use of increased reaction temperatures can also cause the formation of chlorate in the chlorine dioxide composition. Moreover, chlorate can be present in the chlorine dioxide composition because of chlorate contaminations in the chlorite raw material.

From the obtained solution, chlorine dioxide gas can be released and brought through a polyvinylidenedifluoride gas-permeable membrane with a pore size of 1000 nm. Chlorate that is present in mist drops is in this way stopped by the membrane and only chlorine dioxide gas can diffuse through the membrane. In this way, chlorate is avoided to be released in the room to treat while the release of chlorine dioxide is not stopped.

### EXAMPLES 7-15

A room is conditioned at a relative humidity of about 70%. Chlorine dioxide is formulated in an aqueous solution and is released from the aqueous solution. Chlorine dioxide is formulated respectively from chlorite and bisulphate in a molar ratio of 1:1, chlorite and hydrogen chloride in hydrogen chloride in a molar ratio of 1:5 to 2:1, and chlorite and citric acid in a molar ratio of 1:2 to 1:1. The molar ratios have no significant influence on the resulting corrosive behaviour. The release takes place at different flow rates of purging air, i.e. 0.2 m³/h, 1.0 m³/h and 18.0 m³/h. Inox 304 species of 5 cm x 10 cm are exposed to the generated chlorine dioxide gas at a release regime of 720 ppm (vol.)h.

**Table 3. Corrosive behaviour of chlorine dioxide gas with respect to Inox 304 species.**

| Example | activator | purging flow rate (m³/h) | corrosive behaviour a |
|---|---|---|---|
| Example 7 | bisulphate | 18.0 | 3 |
| Example 8 | bisulphate | 1.0 | 3 |
| Example 9 | bisulphate | 0.2 | 3 |
| Example 10 | hydrogen peroxide | 18.0 | 3 |
| Example 11 | hydrogen chloride | 1.0 | 3 |
| Example 12 | hydrogen chloride | 0.2 | 3 |
| Example 13 | citric acid | 18.0 | 3 |
| Example 14 | citric acid | 1.0 | 1 |
| Example 15 | citric acid | 0.2 | 0 |

| | | | |
|---|---|---|---|
| a qualitative determination of corrosive behaviour with respect to an Inox 304 species in which value '3' indicates corrosion of the species, value '2' indicates light corrosion, value '1' indicates corrosion 1 day after the cycle and value '0' indicates no corrosion. | | | |

### EXAMPLES 16-24

The experiments of examples 7-15 were repeated in which a polyvinylidene difluoride gas-selective membrane with a pore size of 10000 nm was arranged in front of the outlet of the reactor. The results of the experiments are shown in Table 4. From the results, it can be derived that the provision of an appropriate gas-selective membrane suppresses the corrosive behaviour of the disinfection treatment significantly and completely, also at high purging flow rates. Hydrogen chloride diffuses through the said gas-permeable membrane, as a result of which the corrosive behaviour of the treatment cannot be suppressed significantly.

**Table 4. Corrosive behaviour of chlorine dioxide gas with respect to Inox 304 species.**

| Example | activator | purging flow rate (m³/h) | corrosive behaviour a |
|---|---|---|---|
| Example 16 | bisulphate | 18.0 | 0 |
| Example 17 | bisulphate | 1.0 | 0 |
| Example 18 | bisulphate | 0.2 | 0 |
| Example 19 | hydrogen chloride | 18.0 | 3 |
| Example 20 | hydrogen chloride | 1.0 | 3 |
| Example 21 | hydrogen chloride | 0.2 | 1 |
| Example 22 | citric acid | 18.0 | 0 |
| Example 23 | citric acid | 1.0 | 0 |
| Example 24 | citric acid | 0.2 | 0 |

| | | | |
|---|---|---|---|
| ^{a} qualitative determination of corrosive behaviour with respect to an Inox 304 species in which value '3' indicates corrosion of the species, value '2' indicates light corrosion, value '1' indicates corrosion 1 day after the cycle and value '0' indicates no corrosion. | | | |

## Claims

1. Method for disinfecting a room with chlorine dioxide gas, comprising the steps:
- generating chlorine dioxide gas in a reaction cell;
- releasing said chlorine dioxide gas from said reaction cell; and
- transferring the released chlorine dioxide gas to the room to treat.

2. Method of claim 1, in which the released chlorine dioxide is brought through the gas-permeable membrane before being released in the room to treat.

3. Method of claim 2, in which said gas-permeable membrane has a pore size smaller than 5.0 µm, preferably smaller than 2.5 µm.

4. Method of any one of the claims 1 to 3, in which said reaction cell comprises an aqueous solution; in which chlorine dioxide gas in said aqueous solution is generated as a result of the reaction of chlorite with an activator; and in which the molar ratio chlorite : activator is lower than 4:1.

5. Method of claim 4, in which said activator is a fluid or a solid at standard pressure and temperature.

6. Method of claim 5, in which said activator is a solid at standard pressure and temperature.

7. Method of any one of the claims 4 to 6, in which said activator in acid form is an acid that does not dissociate in water and an acid gas at standard pressure and temperature.

8. Method of any one of the claims 4 to 7, in which chlorite is activated by said activator in the presence of hydrogen peroxide.

9. Method of claim 8, in which hydrogen peroxide and chlorite are mixed in a molar ratio of about 3:1.

10. Method of any one of the claims 4 to 9, in which said chlorine dioxide gas is released from said aqueous solution by purging the aqueous solution with air.

11. Method of any one of the claims 4 to 10, in which chlorite is dosed in said aqueous environment in an amount of more than 0.5% in weight.

12. Method of any one of the claims 1 to 11, in which the room to treat is previously conditioned at a relative humidity of 50% to 85%.

13. Method of any one of the claims 1 to 12, in which the room to treat is previously conditioned at a temperature of 15°C to 35°C.

14. Method of any one of the claims 1 to 13, in which remaining amounts of chlorine dioxide gas in the room to treat are removed after disinfection.

15. System for disinfecting a room with chlorine dioxide, comprising a reaction cell for formulating and/or storing chlorine dioxide gas, a dispersing unit in fluidic connection with said reaction cell for dispersing the chlorine dioxide gas in said reaction cell in the room to treat and a gas-permeable membrane placed between said reaction cell and said dispersion unit.

16. Use of a method of any one of the claims 1 to 14 for disinfecting a room in which apparatus, such as for example electric and/or electronic devices, are placed.
